# FASCICULE DE BREVET EUROPEEN

(11) **EP 2 398 539 B1**
(45) Date de publication et mention de la délivrance du brevet: **06.06.2018**
(21) Numéro de dépôt: 10707071.6
(22) Date de dépôt: 05.02.2010
(51) Int. Cl.: A61M 16/04

(54) **CANULE TRACHÉALE**
TRACHEAKANÜLE
TRACHEAL CANNULA

(30) Priorité: 20.02.2009 FR 0900779
(43) Date de publication de la demande: 28.12.2011
(73) Titulaire: Bezicot, Eric, 92160 Anthony (FR)
(72) Inventeur: Bezicot, Eric, 92160 Anthony (FR)
(74) Mandataire: Gaillarde, Frédéric F. Ch.
(86) Numéro de dépôt international: PCT/FR2010/000084
(87) Numéro de publication internationale: WO 2010/094849

(56) Documents cités:
- DE-A1- 2 601 623
- FR-A- 1 442 810
- GB-A- 191 328 726
- US-A- 3 606 669

## Description

La présente invention se rapporte à une canule trachéale.

Une canule trachéale est un tube permettant le passage de l'air à travers un orifice pratiqué dans une face antérieure de la trachée après une trachéotomie ou laryngectomie.

Ainsi une canule trachéale vise à permettre la respiration après introduction dans la trachée quand il existe un obstacle dans le larynx.

Une trachéotomie peut être temporaire ou définitive.

Cette canule peut être réalisée dans divers matériaux et pourra être par exemple en matière plastique, métal, silicone, caoutchouc, PVC.

La canule peut être rigide ou souple, droite ou courbée, de longueur et de calibre variable selon le patient et l'utilisation.

Une canule de trachéotomie peut être définitive, et sera alors préférentiellement réalisée à partir d'argent, soit utilisée de manière épisodique et pourra alors être réalisée en caoutchouc, silicone, ...

Alors qu'une canule en argent peut être utilisée plusieurs années, une canule réalisée dans d'autres matériaux peut être amenée à devoir être retirée temporairement en vue de son changement et de son entretien, ou définitivement lorsque la trachéotomie est temporaire.

Un premier changement de canule intervient généralement environ deux jours après l'opération. Des soins de canules nécessitant le retrait de la canule sont nécessaires quotidiennement dans les jours suivant l'opération.

On comprend donc que le retrait de la canule peut être fréquent et qu'il existe un besoin pour faciliter son extraction et sa remise en place.

Plus particulièrement, en cas de soin complet et lorsque la canule doit être retirée assez longtemps, il existe un risque de réduction voire de refermeture du trachéostome. Il existe par conséquent un besoin pour un dispositif permettant de réduire le temps d'intervention au cours duquel la canule est retirée.

Par ailleurs, on notera que la canule devant être introduite dans la trachée, sa manipulation est généralement difficile et ne présente que peu de prise.

De manière générale, pour le confort du patient, le retrait et la remise en place de la canule doit pouvoir s'effectuer le plus rapidement possible.

La présente invention vise à permettre de réduire le temps de retrait et de remise en place d'une canule trachéale et consiste pour ce faire en une canule comprenant un corps tubulaire présentant une première extrémité destinée à être introduite à l'intérieur d'un orifice chirurgical ou naturel d'un patient et une deuxième extrémité destinée à rester à l'extérieur et venir au contact de l'orifice par l'intermédiaire d'un rebord, caractérisée en le que ce rebord présente au moins une languette de préhension orientée vers l' intérieur du rebord. Ainsi, en équipant la canule d'une languette de préhension, la manipulation de la canule lors de son retrait et de sa remise en place s'en trouve grandement facilité, ce qui résulte en une plus grand rapidité d'intervention appréciable pour le patient.

Avantageusement, en ce que la languette est orientée vers l'intérieur du rebord. Il a en effet été constaté que la présence d'une languette ne perturbait pas notablement le passage de l'air à travers la canule. Par ailleurs, ceci permet de conserver le rebord périphérique externe et d'y adapter un filtre ou un nez artificiel, par exemple.

De manière préférentielle, la languette possède une forme arrondie.

Bien évidemment, diverses formes de languette sont envisageables. On pourra citer des languettes de forme sensiblement rectangulaire, triangulaire avec une pointe arrondie, etc.

Avantageusement, la languette est réalisée dans un matériau souple.

Avantageusement encore, la languette est réalisée d'une seule pièce avec la canule, par exemple par moulage.

Selon une première variante de réalisation, la canule est réalisée à partir de silicone.

Selon une deuxième variante de réalisation la canule est réalisée à partir d'une matière plastique de type PVC notamment.

Avantageusement, la deuxième extrémité possède une forme d'entonnoir.

Préférentiellement, la canule est une canule trachéale.

La présente invention sera mieux comprise à l'aide de la description détaillée qui est exposée ci-dessous en regard du dessin annexé dans lequel :
- la figure 1 est une représentation d'une canule trachéale selon l'invention,
- la figure 2 est une représentation schématique en coupe longitudinale de la canule trachéale de la figure 1,
- la figure 3 est une représentation de face de la canule trachéale de la figure 1,
- la figure 4 est une représentation partielle agrandie de la deuxième extrémité de la canule trachéale au niveau de la languette de préhension.

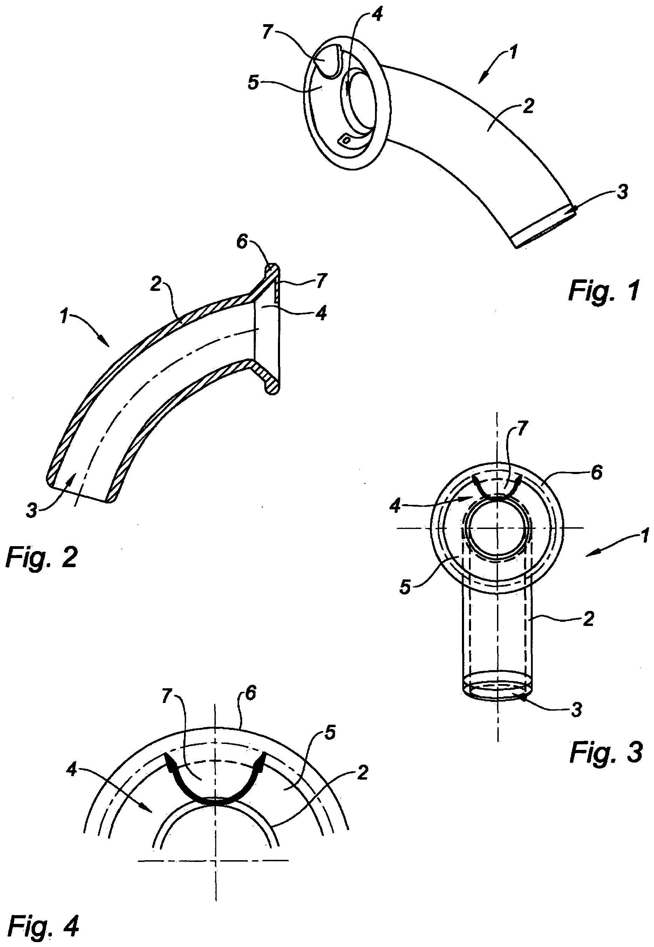

Une canule trachéale 1 telle que représentée sur les figures 1 à 3, comprend un corps tubulaire 2 légèrement courbé réalisé en silicone de qualité médicale.

La canule 1 présente une première extrémité 3 destinée à être insérée à travers un trachéostome, et une deuxième extrémité 4 destinée à rester à l'extérieur et à venir au contact dudit trachéostome.

L'extrémité 4 présente une tête 5 en forme d'entonnoir possédant un bourrelet 6 périphérique servant d'appui et généralement apte à permettre le rattachement d'un nez artificiel, filtre ou autre dispositif.

Conformément à l'invention, le bourrelet 6 est équipé d'une languette 7 de préhension, orientée vers l'intérieur dudit bourrelet 6 périphérique, et présentant une forme sensiblement en demi cercle.

La languette 6 sera préférentiellement réalisée intégralement avec la canule par thermomoulage du silicone.

Ainsi, un utilisateur souhaitant retirer la canule 1 saisira entre se doigts la languette 7 afin d'exercer dessus une traction dans le sens du retrait.

Inversement, lors de la remise en place de la canule 1, la languette facilitera la remise en place finale.

Bien que l'invention ait été décrite avec un exemple particulier de réalisation, il est bien évident qu'elle n'y est nullement limitée et qu'elle comprend tous les équivalents techniques des moyens décrits ainsi que leurs combinaisons si celles-ci entrent dans le cadre de l'invention. On pourra notamment prévoir, au niveau d'une colerette de la canule par exemple, des ouvertures permettant le passage d'un cordon.

## Revendications

1. Canule (1) comprenant un corps (2) tubulaire présentant une première extrémité (3) destinée à être introduite à l'intérieur d'un orifice chirurgical ou naturel d'un patient et une deuxième extrémité (4) destinée à rester à l'extérieur et venir au contact de l'orifice par l'intermédiaire d'un rebord (6), **caractérisée en ce que** le rebord présente au moins une languette de préhension (7) orientée vers l'intérieur du rebord (6).

2. Canule (1) selon la revendication 1, **caractérisée en ce que** la languette (7) possède une forme arrondie.

3. Canule (1) selon la revendication 1 ou 2, **caractérisée en ce que** la languette est réalisée dans un matériau souple.

4. Canule (1) selon l'une quelconque des revendications 1 à 3, **caractérisée en ce que** la languette (7) est réalisée d'une seule pièce avec la canule, par exemple par moulage.

5. Canule (1) selon l'une quelconque des revendications 1 à 4, **caractérisée en ce que** la canule est réalisée à partir de silicone.

6. Canule (1) selon l'une quelconque des revendications 1 à 5, **caractérisée en ce que** la canule est réalisée à partir d'une matière plastique de type PVC notamment.

7. Canule (1) selon l'une quelconque des revendications 1 à 6, **caractérisée en ce que** la deuxième extrémité (4) possède une forme d'entonnoir.

8. Canule (1) selon l'une quelconque des revendications 1 à 7, **caractérisée en ce qu'**il s'agit d'une canule trachéale.

## Patentansprüche

1. Kanüle (1), umfassend einen rohrförmigen Körper (2), welcher ein erstes Ende (3), das dazu vorgesehen ist, in das Innere einer chirurgischen oder natürlichen Öffnung eines Patienten eingeführt zu werden, und ein zweites Ende (4) aufweist, das dazu vorgesehen ist, auf der Außenseite zu bleiben und über einen Flansch (6) mit der Öffnung in Kontakt zu kommen, **dadurch gekennzeichnet, dass** der Flansch mindestens eine Grifflasche (7) aufweist, die zum Inneren des Flansches (6) hin ausgerichtet ist.

2. Kanüle (1) nach Anspruch 1, **dadurch gekennzeichnet, dass** die Lasche (7) eine gerundete Form besitzt.

3. Kanüle (1) nach Anspruch 1 oder 2, **dadurch gekennzeichnet, dass** die Lasche aus einem weichen Material ausgeführt ist.

4. Kanüle (1) nach einem der Ansprüche 1 bis 3, **dadurch gekennzeichnet, dass** die Lasche (7) einstückig mit der Kanüle ausgeführt ist, zum Beispiel durch Gießen.

5. Kanüle (1) nach einem der Ansprüche 1 bis 4, **dadurch gekennzeichnet, dass** die Kanüle aus Silikon ausgeführt ist.

6. Kanüle (1) nach einem der Ansprüche 1 bis 5, **dadurch gekennzeichnet, dass** die Kanüle aus einem Kunststoffmaterial, insbesondere vom Typ PVC, ausgeführt ist.

7. Kanüle (1) nach einem der Ansprüche 1 bis 6, **dadurch gekennzeichnet, dass** das zweite Ende (4) eine Trichterform besitzt.

8. Kanüle (1) nach einem der Ansprüche 1 bis 7, **dadurch gekennzeichnet, dass** es sich um eine Trachealkanüle handelt.

## Claims

1. A cannula (1) comprising a tubular body (2) having a first end (3) intended to be introduced inside a surgical or natural orifice of a patient and a second end (4) intended to remain outside and come into contact with the orifice via a flange (6), **characterized in that** the flange has at least one gripping tab (7) oriented inwardly of the flange (6).

2. The cannula (1) according to claim 1, **characterized in that** the tab (7) has a rounded shape.

3. The cannula (1) according to claim 1 or 2, **characterized in that** the tab is made of flexible material.

4. The cannula (1) according to any one of claims 1 to 3, **characterized in that** the tab (7) is made in one piece with the cannula, for example by a molding operation.

5. The cannula (1) according to any one of claims 1 to 4, **characterized in that** the cannula is made from silicone.

6. The cannula (1) according to any one of claims 1 to 5, **characterized in that** the cannula is made from a plastic material, in particular of the PVC type.

7. The cannula (1) according to any one of claims 1 to 6, **characterized in that** the second end (4) has a funnel shape.

8. The cannula (1) according to any one of claims 1 to 7, **characterized in that** it is a tracheal cannula.
